# FASCICULE DE BREVET EUROPEEN

(11) **EP 3 000 442 B1**
(45) Date de publication et mention de la délivrance du brevet: **11.01.2017**
(21) Numéro de dépôt: 15185866.9
(22) Date de dépôt: 18.09.2015
(51) Int. Cl.: A61F 2/34, A61F 2/46

(54) **ENSEMBLE COMPRENANT UN IMPLANT EN FORME DE CUPULE ET UN PRÉHENSEUR DE CET IMPLANT, ET PROCÉDÉ DE CONSTITUTION DE CET ENSEMBLE**
SET AUS EINEM PFANNENFÖRMIGEN IMPLANTAT UND EINEM GREIFER FÜR DIESES IMPLANTAT, UND VERFAHREN ZUM ERSTELLEN DIESES SETS
ASSEMBLY COMPRISING A CUP-SHAPED IMPLANT AND A GRIPPER OF SAID IMPLANT, AND METHOD FOR FORMING SAID ASSEMBLY

(30) Priorité: 25.09.2014 FR 1459099
(43) Date de publication de la demande: 30.03.2016
(73) Titulaire: Groupe Lepine, 69730 Genay (FR)
(72) Inventeur: PFAIFER, Patrick, 69250 MONTANAY (FR); AMORIM, Cédric, 69580 SATHONAY CAMP (FR); MOUTTON, Nicolas, 69330 MEYZIEU (FR); GROBOST, Jérôme, 72220 Saint Mars D'Outille (FR)
(74) Mandataire: Jeannet, Olivier

(56) Documents cités:
- EP-A1- 0 982 236
- EP-A1- 1 698 306
- DE-U1-202006 010 069
- FR-A1- 2 797 180
- US-A1- 2005 228 394

## Description

La présente invention concerne un ensemble comprenant un implant en forme de cupule et un préhenseur de cet implant. Elle concerne également un procédé de constitution de cet ensemble.

Ledit implant présente une paroi continue formant intérieurement une surface d'articulation lisse ; il peut en particulier s'agir d'un noyau, notamment en céramique, destiné à être placé dans une cupule cotyloïdienne de prothèse de hanche, ou de la cupule cotyloïdienne d'une prothèse de hanche dite "à double mobilité".

Un implant cotyloïdien de prothèse de hanche comprend, comme cela est bien connu, une cupule ayant fréquemment une forme générale sensiblement hémisphérique, qui est destinée à être implantée dans la cavité cotyloïdienne du bassin d'un patient, et un insert en polyéthylène, destiné à être reçu dans la cavité délimitée par la cupule ; selon une possibilité, cet insert délimite lui-même une cavité articulaire sensiblement hémisphérique, destinée à recevoir la tête d'articulation sphérique d'un implant fémoral ; selon une autre possibilité, cet insert est destiné à recevoir un noyau en céramique formant lui-même ladite cavité articulaire.

Lors de la pose de la prothèse, il est nécessaire de manipuler un tel noyau en céramique pour réaliser son assemblage à un insert, ou de manipuler une telle cupule afin de la sortir de son emballage stérile et de l'impacter dans la cavité cotyloïdienne. Actuellement, ces manipulations ne sont guère aisées, particulièrement lorsqu'il s'agit d'une prothèse de hanche dite "à double mobilité", et l'invention a pour objectif de remédier à cet inconvénient, de manière simple et facile d'utilisation.

Il est connu de faciliter la préhension d'une cupule de prothèse de hanche au moyen d'une tête de préhension équipée d'une ventouse, cette ventouse étant appliquée contre la face interne de la cupule et la tête de préhension étant emballée avec la cupule ; après ouverture de l'emballage stérile contenant l'ensemble formé par la tête de préhension et la cupule, cet ensemble est saisi au moyen d'un manche ; après impaction, la ventouse est décollée de la cupule. Bien que cette technique soit avantageuse, elle n'est malheureusement pas applicable à des implants de petite taille, en particulier à des noyaux en céramique.

L'invention a particulièrement pour objectif de remédier à cet inconvénient.

En outre, la publication de demande de brevet N° FR 2 797 180 A1 décrit un ensemble comprenant un implant en forme de cupule, ayant une paroi continue qui délimite une cavité, et un préhenseur de cet implant, ledit préhenseur comprenant :
- une partie d'engagement destinée à être engagée dans la cavité délimitée par l'implant et à venir, dans une position d'engagement dans cette cavité, à proximité immédiate de la paroi de l'implant ; cette partie d'engagement comporte, sur l'ensemble de son pourtour, un joint d'étanchéité venant normalement, dans cette même position d'engagement, en application étanche contre ladite paroi ; et
- une partie de préhension dimensionnée de manière à être apte à être saisie manuellement.

L'ensemble selon ce document antérieur ne permet pas de remédier à l'inconvénient précité.

L'ensemble concerné comprend, de manière connue en soi, un implant en forme de cupule, ayant une paroi continue qui délimite une cavité, et un préhenseur de cet implant, ledit préhenseur comprenant :
- une partie d'engagement destinée à être engagée dans la cavité délimitée par l'implant et à venir, dans une position d'engagement dans cette cavité, à proximité immédiate de la paroi de l'implant ; cette partie d'engagement comporte, sur l'ensemble de son pourtour, un joint d'étanchéité venant normalement, dans cette même position d'engagement, en application étanche contre ladite paroi ; et
- une partie de préhension dimensionnée de manière à être apte à être saisie manuellement.

Selon l'invention,
- le joint d'étanchéité est apte, lorsque l'ensemble est placé en dépression, à légèrement reculer dans le sens radial afin de laisser s'échapper l'air situé entre l'implant et la partie d'engagement ; et
- la partie de préhension est en forme de palette et fait saillie de ladite partie d'engagement.

Le procédé de constitution dudit ensemble comprend les étapes consistant à :
a) utiliser un implant et un préhenseur tels que précités ;
b) insérer ladite partie d'engagement dans la cavité de l'implant, jusqu'à ladite position d'engagement ;
d) réaliser une dépression d'air autour dudit ensemble de manière à faire s'échapper l'air situé entre l'implant et la partie d'engagement ; et
f) ramener ledit ensemble à la pression atmosphérique, de façon à réaliser l'assemblage de l'implant et du préhenseur au moyen de la dépression d'air crées entre cet implant et ce préhenseur.

Ladite dépression d'air permet de créer une liaison entre l'implant et le préhenseur, de sorte que le préhenseur permet de saisir et de manipuler l'implant facilement.

La séparation du préhenseur et de l'implant est réalisée également facilement en inclinant le préhenseur par rapport à l'implant de manière à décoller légèrement un secteur du joint vis-à-vis de la paroi de l'implant, faisant ainsi entrer de l'air entre l'implant et le préhenseur.

De préférence, ledit ensemble comprend un élément de maintien appliqué étroitement sur l'implant et le préhenseur de manière à maintenir le préhenseur dans une position coaxiale à l'implant.

Ce maintien élimine le risque d'inclinaison accidentelle du préhenseur par rapport à l'implant, susceptible de supprimer la liaison réalisée entre le préhenseur et l'implant. De préférence, ledit élément de maintien est un sachet étanche à l'air, dans lequel sont placés l'implant et le préhenseur puis dans lequel est réalisée une dépression d'air de telle sorte que la paroi du sachet épouse la forme de l'implant et du préhenseur.

Un parfait maintien de cet implant et de ce préhenseur est ainsi obtenu.

Le procédé comprend dans ce cas une étape c) entre les étapes b) et d) précitées, consistant à placer l'implant et le préhenseur dans ledit sachet étanche, et une étape e) entre les étapes d) et f) précitées, consistant à sceller ce sachet de manière étanche à l'air.

De préférence, ladite partie d'engagement présente une portion d'extrémité libre en forme de dôme, une portion de base de forme cylindrique et une gorge aménagée entre ces portions, recevant le joint d'étanchéité, la portion de base définissant avec la gorge une arête circulaire vive ayant un diamètre tel que cette arête vient en appui contre la paroi de l'implant, en dessous de l'ouverture de ladite cavité.

La venue de l'arête circulaire vive au contact de la paroi de l'implant définit ainsi précisément ladite position d'engagement, et la forme cylindrique de ladite portion de base fait qu'il existe un interstice entre cette portion de base et la paroi de l'implant, autorisant, lorsqu'il s'agit de séparer l'implant et le préhenseur, l'inclinaison du préhenseur de part et d'autre de la position dans laquelle le préhenseur est coaxial à l'implant.

L'invention sera bien comprise, et d'autres caractéristiques et avantages de celle-ci apparaîtront, en référence au dessin schématique annexé ; ce dessin représente, à titre d'exemple non limitatif, une forme de réalisation préférée de l'ensemble implant-préhenseur-sachet concerné.
La figure 1 est une vue de côté, en coupe transversale et en éclaté, de l'implant en forme de cupule et du préhenseur de cet implant ;
la figure 2 est une vue dudit ensemble similaire à la figure 1, dans une position d'engagement du préhenseur dans une cavité délimitée par l'implant ;
la figure 3 est une vue dudit ensemble similaire à la figure 2, lors de l'introduction de cet ensemble dans le sachet ; et
la figure 4 est une vue dudit ensemble similaire à la figure 3, après mise en dépression du sachet et scellement de ce sachet.

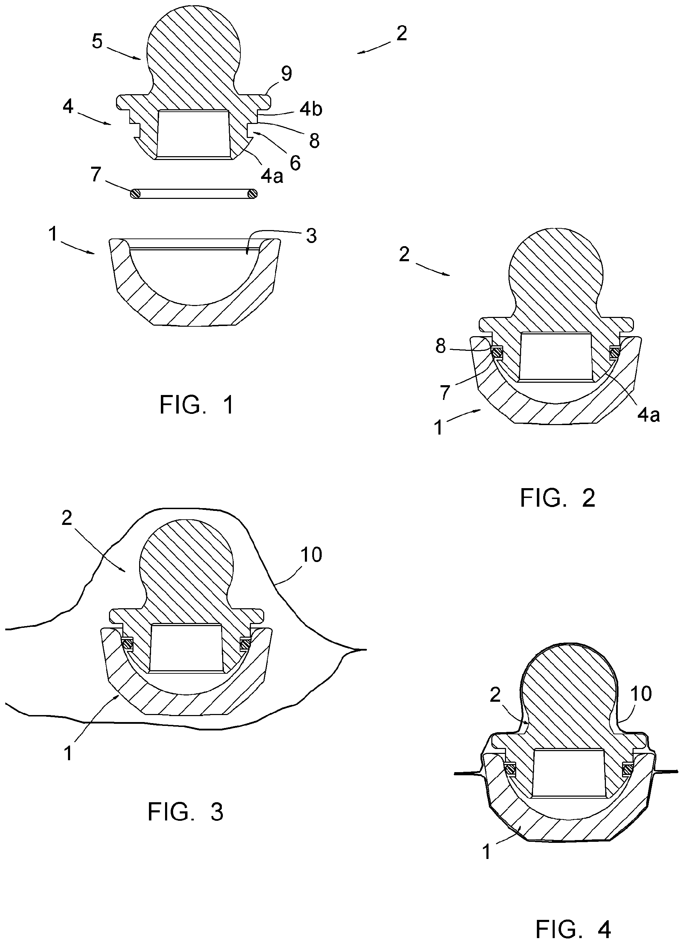

La figure 1 représente un implant 1 en forme de cupule et un préhenseur 2 de cet implant, permettant la manipulation de ce dernier.

L'implant 1 est, dans l'exemple représenté, un noyau en céramique destiné à être placé dans un insert de cupule cotyloïdienne de prothèse de hanche. Il présente une paroi continue formant intérieurement une surface d'articulation lisse, et délimite une cavité 3.

Le préhenseur 2 comprend une partie d'engagement 4 et une partie de préhension 5.

La partie d'engagement 4 présente une portion 4a d'extrémité libre en forme de dôme, une portion 4b de base de forme cylindrique, et une gorge 6 aménagée entre ces portions 4a et 4b, recevant un joint torique d'étanchéité 7.

La portion 4b définit, avec la gorge 6, une arête circulaire vive 8 et a un diamètre tel que cette arête vient en appui contre la paroi de l'implant 1 située en dessous de l'ouverture de la cavité 3, comme visible sur la figure 2. La venue de l'arête 8 au contact de la paroi de l'implant 1 définit ainsi précisément une position d'engagement de la partie 4 dans la cavité 3, et la forme cylindrique de la portion 4b fait qu'il existe un interstice entre cette portion 4b et la paroi de l'implant 1, autorisant une inclinaison du préhenseur 2 de part et d'autre de la position représentée sur la figure 2, dans laquelle le préhenseur 2 est coaxial à l'implant 1.

La profondeur de la gorge 6 et le diamètre du joint 7 sont tels que ce joint fait saillie au-delà de la paroi des portions 4a et 4b lorsqu'il est engagé dans la gorge 6 mais qu'il reste apte à être décollé de la paroi de l'implant 1 lorsque le préhenseur 2 est incliné par rapport à cet implant.

La partie de préhension 5 fait saillie de la partie d'engagement 4 et a la forme d'une palette dimensionnée de manière à être apte à être aisément saisie manuellement.

Le préhenseur 2 comprend également une collerette 9 entre la portion de base 4b et la partie de préhension 5.

L'ensemble formé par l'implant 1 et le préhenseur 2, dans la position d'engagement montrée par la figure 2, est placé dans le sachet 10 dont la paroi est étanche à l'air, notamment en matière synthétique, puis l'intérieur du sachet est mis en dépression d'air de telle sorte que la paroi de ce sachet 10 épouse la forme de l'implant 1 et du préhenseur 2, comme montré sur la figure 4.

Lors de cette mise en dépression, l'air situé entre l'implant 1 et la partie d'engagement 4 s'échappe par suite d'un léger recul radial du joint 7 ; lors de l'ouverture du sachet 10, le retour de l'ensemble à une pression atmosphérique réalise une application du joint 7 contre la paroi de l'implant 1, réalisant une liaison entre cet implant 1 et le préhenseur 2.

Le sachet 10, tant qu'il n'est pas descellé, assure le maintien de l'implant 1 et du préhenseur 2 coaxialement l'un à l'autre, notamment au cours d'un transport de l'ensemble, et ce maintien coaxial assure la réalisation de ladite liaison lorsque le sachet 10 est ouvert.

Comme cela apparaît de ce qui précède, l'invention fournit un ensemble permettant une préhension et une manipulation facile de l'implant 1, notamment lorsque cet implant est de petite taille, et particulièrement lorsqu'il s'agit d'un noyau en céramique destiné à être assemblé à un insert de glissement lui-même destiné à être placé dans une cupule cotyloïdienne de prothèse de hanche.

L'invention a été décrite ci-dessus en référence à une forme de réalisation donnée à titre d'exemple. Il va de soi qu'elle n'est pas limitée à cette forme de réalisation mais qu'elle s'étend à toutes les formes de réalisation couvertes par les revendications ci-annexées.

## Revendications

1. Ensemble comprenant un implant (1) en forme de cupule, ayant une paroi continue qui délimite une cavité (3), et un préhenseur (2) de cet implant (1), ledit préhenseur (2) comprenant :
- une partie d'engagement (4) destinée à être engagée dans la cavité (3) délimitée par l'implant (1) et à venir, dans une position d'engagement dans cette cavité (3), à proximité immédiate de la paroi de l'implant (1); cette partie d'engagement (4) comporte, sur l'ensemble de son pourtour, un joint d'étanchéité (7) venant normalement, dans cette même position d'engagement, en application étanche contre ladite paroi ; et
- une partie de préhension (5) dimensionnée de manière à être apte à être saisie manuellement ;
**caractérisé en ce que** :
- le joint d'étanchéité (7) est apte, lorsque l'ensemble est placé en dépression, à légèrement reculer dans le sens radial afin de laisser s'échapper l'air situé entre l'implant (1) et la partie d'engagement (4) ; et
- la partie de préhension (5) est en forme de palette et fait saillie de ladite partie d'engagement (4).

2. Ensemble selon la revendication 1, **caractérisé en ce qu'**il comprend un élément de maintien (10) appliqué étroitement sur l'implant (1) et le préhenseur (2) de manière à maintenir le préhenseur (2) dans une position coaxiale à l'implant (1).

3. Ensemble selon la revendication 2, **caractérisé en ce que** ledit élément de maintien est un sachet (10) étanche à l'air.

4. Ensemble selon l'une des revendications 1 à 3, **caractérisé en ce que** ladite partie d'engagement (4) présente une portion (4a) d'extrémité libre en forme de dôme, une portion de base (4b) de forme cylindrique et une gorge (6) aménagée entre ces portions, recevant le joint d'étanchéité (7), la portion de base (4b) définissant avec la gorge (6) une arête circulaire vive (8) ayant un diamètre tel que cette arête vient en appui contre la paroi de l'implant (1), en dessous de l'ouverture de ladite cavité (3).

5. Procédé de constitution de l'ensemble selon la revendication 1, **caractérisé en ce qu'**il comprend les étapes consistant à :
a) utiliser un implant (1) et un préhenseur (2) tels que précités ;
b) insérer ladite partie d'engagement (4) dans la cavité (3) de l'implant (1), jusqu'à ladite position d'engagement ;
d) réaliser une dépression d'air autour dudit ensemble de manière à faire s'échapper l'air situé entre l'implant (1) et la partie d'engagement (4) ; et
f) ramener ledit ensemble à la pression atmosphérique, de façon à réaliser l'assemblage de l'implant (1) et du préhenseur (2) au moyen de la dépression d'air crée entre cet implant (1) et ce préhenseur (2).

6. Procédé selon la revendication 5, de constitution de l'ensemble selon la revendication 3, **caractérisé en ce qu'**il comprend une étape c) entre les étapes b) et d) précitées, consistant à placer l'implant (1) et le préhenseur (2) dans ledit sachet étanche (10), et une étape e) entre les étapes d) et f) précitées, consistant à sceller ce sachet (10) de manière étanche à l'air.

## Patentansprüche

1. Anordnung, umfassend ein pfannenförmiges Implantat (1) mit einer kontinuierlichen Wand, die einen Hohlraum (3) begrenzt, sowie einen Greifer (2) für dieses Implantat (1), wobei der Greifer (2) umfasst:
- ein Eingriffsteil (4), das dazu ausgelegt ist, in den Hohlraum (3) einzugreifen, der durch das Implantat (1) begrenzt ist, und in einer Eingriffsposition in diesem Hohlraum (3) in unmittelbarer Nähe zur Wand des Implantats (1) zu gelangen, wobei dieses Eingriffsteil (4) über seinen gesamten Umfang eine Dichtung (7) umfasst, die normalerweise in dieser Eingriffsposition in dichte Anlage gegen die Wand gerät; und
- einen Greifteil (5), der derart dimensioniert ist, dass er dazu ausgelegt ist, manuell erfasst zu werden;
**dadurch gekennzeichnet, dass**:
- die Dichtung (7) dazu ausgelegt ist, dann, wenn die Anordnung unter Unterdruck gesetzt ist, leicht in der radialen Richtung zurückzuweichen, um die Luft entweichen zu lassen, die sich zwischen dem Implantat (1) und dem Eingriffsteil (4) befindet; und
- der Greifteil (5) flügelförmig ist und von dem Eingriffsteil (4) vorsteht.

2. Anordnung nach Anspruch 1, **dadurch gekennzeichnet, dass** sie ein Halteelement (10) umfasst, das eng an dem Implantat (1) und dem Greifer (2) derart angebracht ist, dass es den Greifer (2) in einer zum Implantat (1) koaxialen Position hält.

3. Anordnung nach Anspruch 2, **dadurch gekennzeichnet, dass** das Halteelement ein luftdichter Sack (10) ist.

4. Anordnung nach einem der Ansprüche 1 bis 3, **dadurch gekennzeichnet, dass** der Eingriffsteil (4) einen Bereich (4a) mit einem freien Ende in Kuppelform aufweist, einen Basisbereich (4b) mit zylindrischer Form und eine Kehle (6), die zwischen diesen Bereichen vorgesehen ist, die die Dichtung (7) aufnimmt, wobei der Basisbereich (4b) zusammen mit der Kehle (6) eine scharfe kreisförmige Kante (8) definiert, die einen Durchmesser derart hat, dass diese Kante in Anlage gegen die Wand des Implantats (1) unterhalb der Öffnung des Hohlraums (3) gelangt.

5. Verfahren zur Herstellung der Anordnung nach Anspruch 1, **dadurch gekennzeichnet, dass** es die folgenden Schritte umfasst:
a) Verwenden eines Implantats (1) und eines Greifers (2), wie vorstehend genannt;
b) Einsetzen des Eingriffsteils (4) in den Hohlraum (3) des Implantats (1) bis zur Eingriffsposition;
d) Realisieren eines Luftunterdrucks um die Anordnung herum derart, dass die Luft entweicht, die sich zwischen dem Implantat (1) und dem Engriffsteil (4) befindet; und
f) Bringen der Anordnung auf Atmosphärendruck derart, dass das Zusammenfügen des Implantats (1) und des Greifers (2) mit Hilfe des Luftunterdrucks realisiert wird, der zwischen diesem Implantat (1) und diesem Greifer (2) erzeugt ist.

6. Verfahren nach Anspruch 5 zur Herstellung der Anordnung nach Anspruch 3, **dadurch gekennzeichnet, dass** es einen Schritt c) zwischen den genannten Schritten b) und d) umfasst, der darin besteht, das Implantat (1) und den Greifer (2) in den dichten Sack (10) zu platzieren, sowie einen Schritt e) zwischen den genannten Schritten d) und f), der darin besteht, den Sack (10) luftdicht zu versiegeln.

## Claims

1. Assembly comprising a cup-shaped implant (1) having a continuous wall defining a cavity (3) and a gripper (2) of said implant (1), said gripper (2) comprising:
- an engagement portion (4) intended to be engaged in the cavity (3) delimited by the implant (1) and to come, in a position of engagement in this cavity (3), in the immediate vicinity of the wall of the implant (1); this engagement portion (4) comprises, over the whole of its periphery, a sealing gasket (7) that is normally sealingly applied against said wall in this same engagement position; and
- a gripping part (5) dimensioned so as to be able to be grasped manually;
**characterized in that**:
- the sealing gasket (7) is capable, when the assembly is placed under reduced pressure, of slightly retracting in the radial direction in order to allow air to escape between the implant (1) and the engagement portion (4); and
- the gripping part (5) is in the form of a pallet and projects from said engagement portion (4).

2. Assembly according to claim 1, **characterized in that** it comprises a holding element (10) applied closely to the implant (1) and the gripper (2) so as to hold the gripper (2) in a position coaxial to the implant (1).

3. Assembly according to claim 2, **characterized in that** said holding element is an airtight bag (10).

4. Assembly as claimed in one of claims 1 to 3, **characterized in that** said engagement portion (4) has a dome-shaped free end portion (4a), a cylindrical-shaped base portion (4b) and a groove (6) provided between these portions, receiving the seal (7), the base portion (4b) defining with the groove (6) a sharp circular edge (8) having a diameter such that this edge is capable to bear against the wall of the implant (1), below the opening of said cavity (3).

5. Method of constituting the assembly according to claim 1, **characterized in that** it comprises the steps consisting in:
a) using an implant (1) and a gripper (2) as aforesaid;
b) inserting said engaging portion (4) into the cavity (3) of the implant (1), to said engaging position;
d) providing an air depression around said assembly so as to cause the air between the implant (1) and the engaging portion (4) to escape; and
f) returning said assembly to atmospheric pressure so as to bring obtain the assembly of the implant (1) and the gripper (2) by means of the air depression created between said implant (1) and said gripper (2).

6. Method as claimed in claim 5, for constituting the assembly according to claim 3, **characterized in that** it comprises a step c) between the above-mentioned steps b) and d), consisting in placing the implant (1) and the gripper (2) in said sealed pouch (10), and a step e) between said steps d) and f), consisting in airtightly sealing said pouch (10).
